# EUROPEAN PATENT APPLICATION

(11) **EP 3 225 611 A1**
(43) Date of publication of application: **04.10.2017**
(21) Application number: 16162681.7
(22) Date of filing: 29.03.2016
(51) Int. Cl.: C07C 17/16, C07C 51/60, C07C 57/72

(54) **PREPARATION OF HALIDE PRODUCTS**

(71) Applicant: Nederlandse Organisatie voor toegepast- natuurwetenschappelijk onderzoek TNO, 2595 DA 's-Gravenhage (NL)
(72) Inventor: CROCKATT, Marc, 2595 DA 's-Gravenhage (NL); GEERS, Leonard Fredinand Gerard, 2595 DA 's-Gravenhage (NL); VAN SOMEREN, Rudolf Gijsbertus, 2595 DA 's-Gravenhage (NL); WILES, Charlotte, 6167 RD Geleen (NL)
(74) Representative: V.O.

(57) **Abstract**

The invention is directed to a method for the preparation of an acyl or alkyl halide, comprising, respectively, reacting a carboxylic acid or an alcohol with a halogenation agent in the presence of a base, wherein the reaction is at least partially carried out in a continuous-flow reactor.

## Description

The invention is directed to the preparation of halide products such as acyl or alkyl halides, in particular acyl or alkyl chlorides, from carboxylic acids or alcohols, respectively.

Halide products such as acyl and alkyl halides, in particular acyl and alkyl chlorides, are commonly encountered intermediates in chemical processes. Acyl or alkyl halides are typically prepared by respectively reacting a carboxylic acid or alcohol with a large excess of an halogenation agent. For instance, the preparation of for instance acyl chlorides may be carried out in a batch process by reacting a carboxylic acid with a large excess of thionyl chloride (SOCl₂) which results in - besides the acyl chloride - the gases sulfur dioxide and hydrogen chloride. These gases are allowed to escape the reaction mixture such that the reaction is irreversible and acyl chloride is obtained in high yields (see *e.g.* J. Clayden et al. (2001) Organic Chemistry, Oxford: Oxford University Press. pp. 274-296).

After their preparation, the excess of the halogenation agent is thus removed and the halide product is rapidly employed in a subsequent reaction. The above-described preparation of the halide products is conventionally carried out in batch processes.

Drawback of the batch processes for the preparation of halide products include safety risks (large volumes are typically involved), side-products due to limited control over the reaction conditions in the batch reactor as well as a slow overall throughput. Moreover, the energy input required to distil off excess halogenation agent is also a drawback of conventional reactions.

It is therefore desirable to provide a method wherein none, or at least less, of the above-described drawbacks are encountered.

The present invention is therefore directed at a method for the preparation of an acyl or alkyl halide, comprising reacting a carboxylic acid or an alcohol, respectively, and a halogenation agent in the presence of a base, wherein the reaction is at least partially carried out in a continuous-flow reactor.

Using a continuous-flow reactor is advantageous since it results in high throughput and significantly reduced safety issues due to the relatively small reaction volumes involved when compared to the conventional batch reactors. An additional advantage of the continuous-flow reactor is the facile scalability. It was found to be possible to scale up the reaction from a small continuous-flow reactor (having an internal volume of about 20 µL) by a factor of more than 300 with no significant loss in product quality.

If the halogenation agent reacts with the carboxylic acid or the alcohol, gases are concomitantly produced with the respective acyl or alkyl halide. However, since continuous-flow reactors are typically sealed reactors, these concomitantly produced reaction gases may not escape the reactor and/or reaction mixture. This hampers the reaction from proceeding well and giving the acyl or alkyl halide the desired high yield. The inventors have surprisingly found that this hampering can be limited or even prevented by the presence of the base. Without wishing to be bound by theory, the inventors believe that some of said concomitantly produced gases are complexed to the base.

The halogenation agent typically comprises a halogenated S or P functionality, more in particular an S-X*ₙ* or P-X*ₙ* functionality, wherein X is a F, Cl, Br, I or a combination thereof, and *n* is an integer, which typically has a value of 2 - 5.

When the halogenation agent comprising S-X*ₙ* or P-X*ₙ* reacts with the carboxylic acid or the alcohol, typically a gaseous hydrogen halide (HX) is concomitantly formed with the acyl or alkyl halide. Without wishing to be bound by theory, it is believed that the reaction to the acyl or alkyl halide proceeds better due to the complexation of the base to the hydrogen halide (HX, *e.g.* HCl or HBr) leading to an irreversible reaction.

A particular embodiment of the present invention is the preparation of acyl or alkyl chlorides. Acyl and alkyl chlorides are particularly useful intermediates and used in a variety of commercial processes. To obtain acyl or alkyl chlorides, it is preferred that the halogenation agent is a chlorination agent, preferably a chlorination agent comprising a S-Cl*ₙ* or P-Cl*ₙ* functionality, more preferably a chlorination agent selected from the group consisting of thionyl chloride (SOCl₂), phosphorus trichloride (PCl₃), or phosphorus pentachloride (PCl₅).

Thionyl chloride is most preferred, since particular good results have been obtained with this chlorination agent. The reaction of the carboxylic acid or the alcohol with thionyl chloride results in the concomitant formation of hydrogen chloride (HCl) and sulfur dioxide (SO₂). If the base in accordance with the present invention is not present during this reaction, only very low yields of acyl chloride are obtained (typically less than 5%), possibly because the reaction is reversed due to the presence of HCl and SO₂. It was found that in the presence of 1.1 equivalent (mol/mol) of base relative to the amount of carboxylic acid or the alcohol, the reaction proceeds much better and high yields are obtained (typically more than 90%). If the above-described theory indeed applies, this favorable result is surprising since it suggests that it is sufficient to only complex and therewith remove the HCl, while removal of the SO₂ is not required to obtain an irreversible reaction.

The molar ratio of the base to the carboxylic acid or the alcohol preferably ranges between 0.1 and 10, preferably between 0.5 and 5, more preferably between 1 and 3, most preferably about 1 since this gave particularly good results. The fact that the ratio of about 1 gave good results, supports the theory that the base complexes with the liberated hydrogen halide.

It may be appreciated that in the context of the present invention, molar ratio is expressed as the molar ratio of the reagents as present in the reaction mixture before the start of the reaction. The desired molar ratio of the reagent can for instance be set by adapting the concentration of one or more of the reagents when a solvent is used and/or by varying the feeding rate of one or more of the reagents to the continuous-flow reactor.

The preparation of the acyl or alkyl halide in accordance to the present invention is typically carried out in a solvent, in particular an organic solvent since aqueous solvent tend to react with either the halogenation agent or the acyl or alkyl halide products. As such, the base typically comprises an organic base. Applying the above-described theory on the function of the base and given the high acidity of hydrogen halides, any organic base generally suffices. However, particularly good results may be obtained with a non- or weak-nucleophilic organic base that does not, or at least to a very limited extent, react with the acyl or alkyl halide product. In particular bases comprising non-nucleophilic organic amine, for instance trialkylamines such as trimethyl amine, triethyl amine, diisopropylethyl amine, *N*-methyl morpholine, *N*-methyl piperidene, 1,4-dimethylpiperazine, or pyridine, 4-(dimethylamino)pyridine, 1,5-diazabicyclo[4.3.0]non-5-ene, 1,8-diazabicyclo[5.4.0]undec-7-ene, 1,4-diazabicyclo[2.2.2]octane, 2,6-lutidine and their solid supported analogues. In a particular embodiment, the base is immobilized on a solid support. This solid support may be immobilized in the continuous-flow reactor, for instance on the internal wall of the reactor that is in contact with the reaction mixture (*e.g*. packed bed).

In another particular embodiment, the base may be regenerated after the reaction; the base complexed to the hydrogen halide (*i.e*. spent base) may be converted into the base as was present before the reaction. In case the base is immobilized in the reactor, the spent base may be generated by flushing the reactor with a liquid that comprises a second base to remove the hydrogen halide. In case the base (and the spent base) flows with the other reagents through the continuous-flow reactor, the base may for instance be regenerated by using an ion exchange column.

An additional drawback of the conventional methods in batch, is that these usually rely on a large excess of halogenation agent is that this agent typically needs to be removed (*e.g*. by distillation) after the reaction and before the next reaction with the acyl halide or alkyl halide product is carried out. The removal of the halogenation agent requires additional energy and time to obtain the acyl or alkyl halide. It is therefore preferred that substantially all the halogenation agent is converted into the acyl or alkyl halide.

The inventors found that, in contrast to in the conventional batch processes, a large excess of the halogenation agent is not required. Advantageously, the molar ratio of the halogenation agent to the carboxylic acid or the alcohol may range between 0.1 and 10, preferably between 0.5 and 5, more preferably between 1 and 3, most preferably about 1. Even when about 1 equivalent (*e.g*. about 1.01 equivalent) of the halogenation agent relative to the carboxylic acid or the alcohol is used, high yields of the acyl or alkyl halide (*e.g*. more than 90%) can be obtained.

The reaction is preferably carried out at an elevated pressure, preferably at more than 1.5 bar, more preferably more than 4 bar, most preferably about 8 bar. Usually the pressure is less than 10 bar. The pressure may be regulated by using a back-pressure regulator on the continuous-flow reactor. The elevated pressure generally assist in maintaining a steady flow in the continuous-flow reactor by keeping any concomitantly formed gasses (*e.g.* SO₂) in solution. The method however, may also be carried out at about atmospheric pressure.

It is preferred that the reaction is carried out at a temperature of 10 to 100 °C, preferably 15 to 50 °C, more preferably 20 to 45 °C, most preferably about 35°C, since good results in terms of yield and required reaction time were obtained at these temperature ranges.

The carboxylic acid of the present invention may be any carboxylic acid. Similarly, the alcohol of the present invention may be any organic alcohol. In fact, a particular advantage of using the continuous-flow reactor in accordance with the present invention is that mild conditions can be used (*e.g.* only 1 equivalent of halogenation agent vis-à-vis the carboxylic acid or the alcohol), distillation after the halogenation reaction, *e.g.* in between the halogenation reaction and the following reaction is not required, a high overall throughput (fast reaction times) is obtained, and the residence time (in the reactor and between reaction steps) is minimized, limiting the residence time distribution, and preventing unnecessary degradation of the sensitive halide compounds. As such, relatively unstable acyl and/or alkyl halides can be obtained, which are usually inaccessible by using the convention batch-wise processes for the preparation of acyl and/or alkyl halides. The present invention is therefore very suitable for the preparation of acyl and/or alkyl halide comprising intermediates that may be further used in processes for the preparation of pharmaceuticals or other highly-functionalized compounds.

A further aspect of the present invention is a method for producing an organic compound, comprising the preparation of the acyl or alkyl halide in a method according to the present invention, followed by further reacting the acyl or alkyl halide. An advantage of the present invention is that after the formation of the acyl or alkyl halide no elaborate intermediate purification (such as distillation) is required before the acyl or alkyl halide is reacted further. Although in certain cases, it may be advantageous to remove the salt of the base (HX), this is not considered an elaborate intermediate purification. In a preferred embodiment of the method for producing the organic compound, the acyl or alkyl halide is thus reacted further without any intermediate purification.

A possible follow-up reaction with the acyl halide as intermediate is a Friedel-Craft acylation with AlCl₃. For this follow-up reaction, using only 1 equivalent of halogenation agent vis-à-vis the carboxylic acid such that substantially all the halogenation agent is converted into the acyl halide, is particularly favorable since the presence of the halogenation agent in the Friedel Craft acylation generally leads to undesired side reactions.

The continuous-flow reactor is known as a reactor for carrying out reactions by way of continuous processing. Other commonly used terms for continuous-flow reactors are continuous reactor or flow reactor. Examples of continuous-flow reactors include a continuously-stirred tank reactor (CSTR), a tube reactor and combinations thereof. A combination of reactors can be arranged in a parallel and/or in a serial fashion.

Preferably a continuous-flow tube reactor is used. Reactors that are particularly favorable are for instance those commercially available from Chemtrix B.V. (Geleen, the Netherlands) under the tradenames Labtrix™ (typically used for laboratory method development using glass micro reactors), KiloFlow™ (typically used for scaling to grams/kilograms using glass meso reactors) and Plantrix™ (typically used for ton scale production using ceramic (SiC) reactors).

In a preferred embodiment of the present invention, before reacting the carboxylic acid or the alcohol and the halogenation agent, a first stream comprising the halogenation agent and a second stream comprising the carboxylic acid or the alcohol and the base are provided. Subsequently, the first liquid stream is contacted with the second liquid stream in the continuous-flow reactor.

Although it may be preferred that all reagents (*i.e*. the carboxylic acid or the alcohol, the halogenation and the base) are dissolved in a solvent, the method also works well when the second stream comprises a suspension of at least part of the carboxylic acid or the alcohol, *i.e*. not all carboxylic acid or all alcohol is dissolved in the solvent. A suspension of at least part of the carboxylic acid may for instance be applicable in case the carboxylic acid is not well soluble in the used solvent (*e.g*. because the carboxylic acid is a zwitter-ion). It was found that the suspension does not result in undesirable clotting of the continuous-flow reactor. Moreover good yields of the acyl halide were obtained, despite that at least part of the carboxylic acid did not dissolve in the solvent.

A further aspect of the present invention is the use of a continuous-flow reactor for the preparation of the acyl or alkyl halide from the carboxylic acid or the alcohol respectively, preferably by a method as described herein above.

For the purpose of clarity and a concise description features are described herein as part of the same or separate embodiments, however, it will be appreciated that the scope of the invention may include embodiments having combinations of all or some of the features described.

The invention may be illustrated by the following examples.

### Comparative Example 1: synthesis of 4-methoxybenzoyl chloride without a base

A 0.2625M solution of thionyl chloride (19 µL) in tetrahydrofuran (1 mL) was prepared and loaded into a syringe. A 0.25 M solution of 4-methoxybenzoic acid (38 mg) in tetrahydrofuran (1 mL) was prepared and loaded into a separate syringe. These two solutions were then introduced to a Labtrix™ continuous-flow microreactor (available from Chemtrix BV (Geleen, the Netherlands)) in a 1:1 ratio at a temperature of 20 °C and after the appropriate residence time the reaction mixture was analyzed by HPLC. This showed a conversion to 4-methoxybenzoyl chloride of 2.4% after 3 minutes and 5.2% after 10 minutes of residence time, with the remaining material being unreacted 4-methoxybenzoic acid.

### Example 1: synthesis of 4-methoxybenzoyl chloride in the presence of a base

A 0.2625M solution of thionyl chloride (19 µL) in dichloromethane (1 mL) was prepared and loaded into a syringe. A solution of 0.25M 4-methoxybenzoic acid (38 mg) and 0.275 M triethylamine (38.4 µL) in dichloromethane (1 mL) was prepared and loaded into a separate syringe.

These two solutions were then introduced to a microreactor in a 1:1 ratio at a temperature of either 0 °C, 20 °C or 35 °C and after the appropriate residence time the reaction mixture was analyzed by HPLC. This showed the conversions to 4-methoxybenzoyl chloride (with the remaining material being unreacted 4-methoxybenzoic acid) as indicated in table 1.

**Table 1**

| **Residence time (sec)** | | **Yield (%)** | |
|---|---|---|---|
| | **20 °C** | **35 °C** | **0°C** |
| 300 | 90.7 | 98.9 | 67.2 |
| 200 | 93.3 | 97.1 | |
| 150 | 91.0 | 97.8 | |
| 100 | 95.4 | | |

## Claims

1. Method for the preparation of an acyl halide or alkyl halide, comprising reacting a carboxylic acid or an alcohol, respectively, with a halogenation agent in the presence of a base, wherein the reaction is at least partially carried out in a continuous-flow reactor.

2. Method according to claim 1, wherein the molar ratio of the base to the carboxylic acid or the alcohol ranges between 0.1 and 10, preferably between 0.5 and 5, more preferably between 1 and 3, most preferably about 1.

3. Method according to any of the previous claims, wherein the base comprises an organic base, preferably a non-nucleophilic organic base, more preferably a non-nucleophilic organic amine, most preferably a trialkylamine or pyridine.

4. Method according to any of the previous claims, wherein the base comprises a compound selected from the group consisting of trimethyl amine, triethyl amine, diisopropylethyl amine, *N*-methyl morpholine, N-methyl piperidene, 1,4-dimethylpiperazine pyridine, 4-(dimethylamino)pyridine, 1,5-diazabicyclo[4.3.0]non-5-ene, 1,8-diazabicyclo[5.4.0]undec-7-ene, 1,4-diazabicyclo[2.2.2]octane, 2,6-lutidine and their solid supported analogues.

5. Method according to any of the previous claims, wherein the molar ratio of the halogenation agent to the carboxylic acid or the alcohol ranges between 0.1 and 10, preferably between 0.5 and 5, more preferably between 1 and 3, most preferably about 1.

6. Method according to any of the previous claims, wherein the halogenation agent comprises a S-X₂ or P-X₂ functionality, wherein X is a F, Cl, Br, I or a combination thereof.

7. Method according to any of the previous claims, wherein the halogenation agent is a chlorination agent, preferably a chlorination agent comprising a S-Cl or P-Cl functionality, more preferably a chlorination agent selected from the group consisting of thionyl chloride (SOCl₂), phosphorus trichloride (PCl₃), or phosphorus pentachloride (PCl₅).

8. Method according to any of the previous claims, wherein reacting the carboxylic acid or the alcohol with the halogenation agent is carried out at an elevated pressure, preferably at more than 1.5 bar, more preferably more than 4 bar, most preferably about 8 bar.

9. Method according to any of the previous claims, wherein reacting the carboxylic acid or the alcohol and the halogenation agent is carried out at a temperature of -10 to 100 °C, preferably 0 to 50°C, more preferably 20 to 45 °C, most preferably about 35°C.

10. Method according to any of the previous claims, wherein the method comprises before reacting the carboxylic acid or the alcohol with the halogenation agent, a step of providing a first stream comprising the halogenation agent and a second stream comprising the carboxylic acid or the alcohol and the base, followed by contacting the first liquid stream with the second liquid stream in the continuous-flow reactor.

11. Method according to claim 10, wherein the second stream comprises a suspension of at least part of the carboxylic acid or the alcohol.

12. Method according to any of the previous claims, wherein substantially all the halogenation agent is converted into the acyl or the alkyl halide.

13. Use of a continuous-flow reactor for the preparation of an acyl halide from a carboxylic acid, or an alkyl halide from an alcohol.

14. Method for producing an organic compound, comprising the preparation of the acyl or alkyl halide in a method according to any of claims 1-12, followed by further reacting the acyl or alkyl halide.
